# EUROPEAN PATENT SPECIFICATION

(11) **EP 3 164 490 B1**
(45) Date of publication and mention of the grant of the patent: **19.09.2018**
(21) Application number: 15747581.5
(22) Date of filing: 02.07.2015
(51) Int. Cl.: C12N 15/115

(54) **DNA APTAMER RECOGNISING ARGININE TAG AND USE THEREOF**
ARGININ-TAG ERKENNENDES DNA-APTAMER UND VERWENDUNG DAVON
APTAMÈRE D'ADN RECONNAISSANT UNE ÉTIQUETTE ARGININE ET SON UTILISATION

(30) Priority: 02.07.2014 PL 40873514
(43) Date of publication of application: 10.05.2017
(73) Proprietor: Uniwersytet Jagiellonski, 31-007 Krakow (PL)
(72) Inventor: BARTNICKI, Filip, PL-30-868 Kraków (PL); KOWALSKA, Ewa, PL-31-422 Kraków (PL); PELS, Katarzyna, PL-38-322 Luzna (PL); STRZALKA, Wojciech, PL-30-373 Kraków (PL)
(74) Representative: BRANDPAT Patent and Trademark Attorneys Chlebicka Czyz Galazkiewicz Ziolkowski Professional Partnership
(86) International application number: PCT/PL2015/050027
(87) International publication number: WO 2016/003302

(56) References cited:
- WO-A2-2005/024042
- US-A1- 2005 283 003
- FUCHS STEPHEN M ET AL: "Polyarginine as a multifunctional fusion tag", PROTEIN SCIENCE, WILEY, US, vol. 14, no. 6, 1 June 2005 (2005-06-01), pages 1538-1544, XP002428590, ISSN: 0961-8368, DOI: 10.1110/PS.051393805
- K Harada ET AL: "Identification of two novel arginine binding DNAs", The EMBO journal, 1 December 1995 (1995-12-01), pages 5798-5811, XP055215514, ENGLAND Retrieved from the Internet: URL:http://www.ncbi.nlm.nih.gov/pubmed/884 6773 [retrieved on 2015-09-23]
- BAYER T S ET AL: "ARGININE-RICH MOTIFS PRESENT MULTIPLE INTERFACES FOR SPECIFIC BINDING BY RNA", RNA, COLD SPRING HARBOR LABORATORY PRESS, US, vol. 11, no. 12, 1 January 2005 (2005-01-01), pages 1848-1857, XP009065545, ISSN: 1355-8382, DOI: 10.1261/RNA.2167605
- HIROYUKI FURUSAWA ET AL: "Arginine Arrangement of Bacteriophage [lambda] N-Peptide Plays a Role as a Core Motif in GNRA Tetraloop RNA Binding", CHEMBIOCHEM, vol. 15, no. 6, 14 April 2014 (2014-04-14) , pages 865-871, XP055215357, ISSN: 1439-4227, DOI: 10.1002/cbic.201300809

## Description

The presented invention is a unique oligonucleotide sequence of a single-stranded DNA aptamer recognising arginine tag and its application.

The development of contemporary molecular biology, biotechnology or medicine depends, among others, on the access to high-quality protein preparations. A vast majority of them are recombinant proteins, which are obtained through production in properly prepared prokaryotic or eukaryotic cells. Quick and easy isolation procedure of the produced protein can be provided by adding a fusion tag to its amino acid sequence which enables purification of the desired protein using affinity chromatography. Among the different types of affinity chromatography used to obtain recombinant proteins, the systems based on binding, for example, histidine tag to a resin containing nickel ions, glutathione S-transferase to a resin with bound glutathione or maltose-binding protein to a resin with dextrin, can be mentioned.

WO 2005/024042 A1 discloses a method for obtaining a DNA aptamer having high affinity to a target molecule containing polyhistidine tag.

US 2005/283003 provides a methods and materials for the oriented attachment of proteins and nucleic acids an atomically smooth surface. A protein can be chemically conjugated to the arginine tag or fused to the amino or carboxyl terminus of the arginine tag. Where the protein is fused, the protein can be recombinantly expressed as a fusion protein with the arginine tag.

DNA aptamers are defined as single-stranded deoxyribonucleic acid molecules about 40-100 nucleotides long, which have an ability to bind to a particular molecular target with high specificity and affinity. Thanks to rich secondary and tertiary structures of single-stranded DNA, it is possible to obtain
optimal match between the selected aptamer and the specific molecular target.

The aim of the invention is to provide a new method of identification of molecules containing an arginine tag (further referred to as "Arg tag"), especially through the provision of a new molecule with high affinity for a molecular target containing the arginine tag, that is any molecule (e.g.: peptide, protein, glycoprotein, DNA derivative modified with linked peptide) containing several successive arginine residues.

Unexpectedly, it turned out that the - aim described above can be achieved using this invention.

The subject of the invention is a DNA aptamer showing affinity for an Arg tag and containing a nucleotide sequence: 5'-CTTTGTAATTGGTTCTGAGTTCCGTTGTGGGAGGAACATG -3' (Sequence Id. No. 1), subsequently also referred to as 24-10 aptamer.

Certain variability of DNA sequences is acceptable, on condition that their affinity for the molecules containing an Arg tag is preserved, which enables selective purification of Arg-tagged proteins from a mixture of several proteins. The scope of the invention particularly encompasses a variant of the above-mentioned sequence, which differs by one purine or pyrimidine base.

Another subject of the invention is the use of 24-10 aptamer with the sequence described above to produce molecules with affinity for molecular targets containing Arg tag, especially:
- to detect molecular targets containing Arg tag,
- to bind molecular targets containing Arg tag,
- to purify molecular targets containing Arg tag,
- to analyse the concentration of molecular targets containing Arg tag.

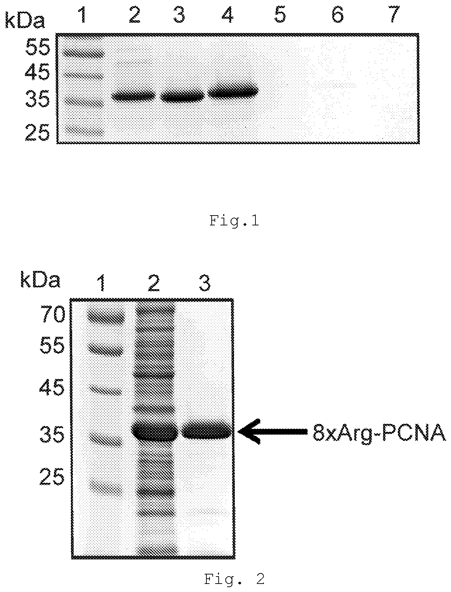

In order to explain better the nature of the invention defined above, the following figures were added to this description: Figure 1 depicts binding between the protein containing the arginine tag and the developed aptamer. Lanes: 1) protein marker, 2) 8xArg-PCNA protein used in the experiment, 3) PCNA protein used in the experiment, 4) protein preparation recovered from the resin containing 24-10 aptamer, which had previously been incubated with 8xArg-PCNA protein, 5) protein preparation recovered from the resin containing 24-10 aptamer, which had previously been incubated with PCNA protein, 6) protein preparation recovered from the resin containing the reference aptamer, which had previously been incubated with 8xArg-PCNA protein, 7) protein preparation recovered from the
resin containing the reference aptamer, which had previously been incubated with PCNA protein. The samples were separated in 12% denaturing polyacrylamide gel and then stained using Coomassie Brilliant Blue R-250.

Figure 2 shows the purification process of the protein containing 8xArg tag from the protein extract prepared from *E. coli* cells. Lanes: 1) protein marker, 2) 40 µg of total protein extract prepared from *E. coli* cells with overexpression of 8xArg-PCNA, 3) 8xArg-PCNA protein purified using chromatographic resin containing bound 24-10 aptamer. The samples were separated in 12% denaturing polyacrylamide gel and then stained using Coomassie Brilliant Blue R-250.

Moreover, this description has been completed with examples of implementation given below. They should not be identified with a full scope of the invention defined above.

Example 1. Obtaining DNA aptamers with affinity for the Arg tag.

As a result of research aimed at developing a DNA molecule showing affinity for molecular targets containing Arg tag, it has been established that DNA aptamer of the following sequence: 5'- CTTTGTAATTGGTTCTGAGTTCCGTTGTGGGAGGAACATG -3' (SEQ ID. No. 1) has strong affinity for such targets.

According to the invention, DNA molecule can be obtained using any routine method of DNA synthesis known to the specialist.

The oligonucleotide molecule was obtained using solid phase chemical synthesis technique (Zlatev, I., Manoharan, M., Vasseur, J.-J. and Morvan, F. Solid-Phase Chemical Synthesis of 5'-Triphosphate DNA, RNA, and Chemically Modified Oligonucleotides. Current Protocols in Nucleic Acid Chemistry. 2012; 50:1.28.1-1.28.16.)

The obtained 521 micrograms of the 24-10 aptamer molecule of 40 nucleotides, with the sequence defined above, was verified using HPLC method.

The DNA molecule containing the sequence compliant with the invention can be additionally modified, especially when coupled to known dyes (ex. fluorescent dyes) or other molecules (ex. biotin).

For example, biotinylated 24-10 aptamer was obtained in the respective amount of 474 micrograms using the method of automated synthesis of 5'-biotinylated oligonucleotides. (Pon, R.T. A Long Chain Biotin Phosphoramidite Reagent for The Automated Synthesis of 5'-Biotinylated Oligonucleotides, Tetrahedron Lett.1991; 32: 1715-1718). The obtained product was verified using the HPLC method.

Example 2. Binding of the Arg-tagged protein to chromatographic resin containing the aptamer according to the invention.

10 µl of 50% agarose bead coupled to streptavidin was placed in a 1.5 ml eppendorf tube and washed with distilled water. It was then washed three times with a BW buffer (17.5 g/L NaCl; 50 mM phosphate buffer NaH₂PO₄/Na₂HPO₄; 0.1% (v/v) Tween 20; pH 7.5). The resin was then suspended in 300 µl of the BW buffer and 20 µg of 5'-biotinylated 24-10 aptamer or a reference aptamer with the (ACTG)x7-ACGAGGAACATG sequence was added. The samples were incubated at room temperature for 1h. After the incubation, the resin was washed three times with the BW buffer followed by two washes with an AS buffer (137mM NaCl; 12.7mM KCl; 10mM Na₂HPO₄; 2mM KH₂PO₄; 5mM MgCl₂; 0.1% (v/v) Tween 20; pH 7.5). After the last washing, the resin with the bound aptamer was suspended in 300 µl of the AS buffer, and the human recombinant PCNA protein was added to achieve a final concentration of 0.13 mg/ml. In this experiment, two variants of the PCNA protein were used: a) with 8xArg-tag and b) without 8xArg-tag. The resin was incubated at room temperature for 1h. After the incubation, it was washed four times with the AS buffer. Then, a GLB buffer (50 mM Tris-HCl, 2% SDS (w/v), 2% bromophenol blue (w/v), 10% glycerol (v/v), 200 mM β-mercaptoethanol, pH 6.8) was added to the resin and incubated for 5 min at 95°C. The obtained sample was subjected to denaturing poliacrylamide gel electrophoresis (SDS-PAGE, Laemmli method) in 12% polyacrylamide gel. After separation, the proteins were stained using Coomassie Brilliant Blue R-250 dye. The results are shown in Figure 1.

Example 3. Purification of Arg-tagged protein from a protein cell extract.

10 µl of 50% agarose resin coupled to streptavidin was placed in a 1.5 ml eppendorf tube and washed with distilled water. It was then washed three times with the BW buffer (0.3M NaCl; 50 mM phosphate buffer NaH₂PO₄/Na₂HPO₄; 0.1% (v/v) Tween 20; pH 7.5). The resin was then suspended in 300 µl of the BW buffer, and 20 µg of 5'-biotinylated 24-10 aptamer was added. The samples were incubated at room temperature for 1h. After the incubation, the resin was washed three times with the BW buffer, followed by two washes with the AS buffer (137mM NaCl; 12.3mM KCl; 10mM Na₂HPO₄; 2mM KH₂PO₄; 5mM MgCl₂; 0.1% (v/v) Tween 20; pH 7.4). After the last washing, the resin with the bound aptamer was suspended in 300 µl of the AS buffer, and the protein extract prepared from *E. coli* cells containing human recombinant 8xArg-PCNA protein was added to achieve a final total protein concentration of 2.0 mg/ml. The resin was incubated at 4°C for lh. After the incubation, it was washed four times with the AS buffer. Then, the 8xArg-PCNA protein was eluted from the resin using an elution buffer (50mM Tris, 500mM NaCl, 300mM guanine hydrochloride, pH 7.5). The preparation composed of the eluted protein was mixed with a GLB buffer (50 mM Tris, 2% SDS (w/v), 2% bromophenol blue (w/v), 10% glycerol (v/v), 200 mM β-mercaptoethanol, pH 6.8) and incubated for 5 min at 95°C. The obtained sample was subjected to denaturing poliacrylamide gel electrophoresis (SDS-PAGE, Laemmli method) in 12% polyacrylamide gel. After separation, the proteins were stained using Coomassie Brilliant Blue R-250 dye. The results are shown in Figure 2.

## Claims

1. DNA aptamer containing a nucleotide sequence presented as SEQ ID. No. 1, or a variant thereof which differs by one purine or pyrimidine base, showing affinity for an Arg tag.

2. Use of the oligonucleotide containing the sequence according to claim 1 in the production of molecules with affinity for molecular targets containing Arg tag, especially:
- to detect molecular targets containing Arg tag,
- to bind molecular targets containing Arg tag,
- to purify molecular targets containing Arg tag,
- to analyse the concentration of molecular targets containing Arg tag.

## Patentansprüche

1. DNA-Aptamer, welches eine Nucleotidsequenz, die als SEQ ID. No. 1 vorliegt, oder eine Variante davon, welche sich durch eine Purin- oder Pyrimidinbase unterscheidet, enthält, welches Affinität zu einem Arg-Tag zeigt.

2. Verwendung des Oligonucleotids, welches die Sequenz nach Anspruch 1 enthält, bei der Herstellung von Molekülen mit Affinität zu molekularen Targets, welche das Arg-Tag enthalten, insbesondere:
- zum Erkennen der das Arg-Tag enthaltenden molekularen Targets,
- zum Binden der das Arg-Tag enthaltenden molekularen Targets,
- zum Reinigen der das Arg-Tag enthaltenden molekularen Targets,
- zum Analysieren der Konzentration der das Arg-Tag enthaltenden molekularen Targets.

## Revendications

1. Aptamère d'ADN contenant une séquence de nucléotides présentée comme SEQ ID. No. 1, ou une variante de celle-ci qui diffère d'une base de purine ou de pyrimidine, montrant une affinité pour une étiquette Arg.

2. Utilisation de l'oligonucléotide contenant la séquence selon la revendication 1 dans la production de molécules ayant une affinité pour des cibles moléculaires contenant une étiquette Arg, en particulier :
- pour détecter des cibles moléculaires contenant une étiquette Arg,
- pour lier des cibles moléculaires contenant une étiquette Arg,
- pour purifier des cibles moléculaires contenant une étiquette Arg,
- pour analyser la concentration de cibles moléculaires contenant une étiquette Arg.


### SEQUENCE LISTING

<110> Uniwersytet Jagielloñski
<120> DNA aptamer recognising arginine tag and use thereof
<130> PZ/2548/RW/PCT
<160> 2
<170> PatentIn version 3.5
<210> 1
   <211> 40
   <212> DNA
   <213> artificial
<220>
   <223> 24-10 aptamer
<400> 1
   ctttgtaatt ggttctgagt tccgttgtgg gaggaacatg 40
<210> 2
   <211> 40
   <212> DNA
   <213> artificial
<220>
   <223> reference aptamer
<400> 2
   actgactgac tgactgactg actgactgac gaggaacatg 40